# FASCICULE DE BREVET EUROPEEN

(11) **EP 2 571 388 B1**
(45) Date de publication et mention de la délivrance du brevet: **04.06.2014**
(21) Numéro de dépôt: 11728307.7
(22) Date de dépôt: 19.05.2011
(51) Int. Cl.: A42B 3/18, A61F 9/02

(54) **DISPOSITIF POUR ÉCARTER -ET MAINTENIR ÉCARTÉ- DU VISAGE UN MASQUE DE PROTECTION OCULAIRE MAINTENU PAR UN BANDEAU ÉLASTIQUE (TYPE SKI OU MOTOCROSS) LORSQU'IL EST PORTÉ SUR UN CASQUE**
VORRICHTUNG ZUR TRENNUNG UND BEWAHRUNG EINES ABSTANDES ZWISCHEN DEM GESICHT EINER PERSON UND EINER MIT EINEM ELASTISCHEN BAND BEFESTIGTEN AUGENSCHUTZBRILLE (SKI- ODER MOTOCROSSBRILLE)
DEVICE FOR SEPARATING, AND MAINTAINING A DISTANCE BETWEEN, A PERSON'S FACE AND A PROTECTIVE EYE MASK HELD IN PLACE BY AN ELASTIC STRAP (SKI OR MOTOCROSS TYPE)

(30) Priorité: 21.05.2010 FR 1002148
(43) Date de publication de la demande: 27.03.2013
(73) Titulaire: Airflaps, 75011 Paris (FR)
(72) Inventeur: GISQUIÈRE, Serge, 84210 Pernes les Fontaines (FR)
(74) Mandataire: Verriest, Philippe
(86) Numéro de dépôt international: PCT/FR2011/000303
(87) Numéro de publication internationale: WO 2011/144827

(56) Documents cités:
- EP-A2- 1 702 596
- WO-A1-01/89333
- DE-A1-102005 058 393
- US-A- 4 686 712
- US-A- 5 642 178
- US-A1- 2008 034 480

## Description

La présente invention concerne un dispositif pour écarter -et maintenir écarté- du visage un masque de protection oculaire maintenu par un bandeau élastique (type ski ou moto-cross) lorsque celui-ci est porté sur un casque.

Ce dispositif est inédit car n'ayant pas d'équivalent à ce jour. Le document WO 0189333 décrit un dispositif tel que connu dans l'art antérieur.

Deux raisons essentielles ont motivé son invention :
La première intervient lorsque l'on retire son casque. Pour optimiser leur efficacité, les masques de protection épousent au mieux la forme du visage grâce au bandeau élastique et aux mousses d'étanchéité. L'enlèvement du casque est dès lors très inconfortable -voire douloureux ou carrément impossible- sans que son porteur se soit préalablement débarrassé dudit masque. Le dispositif décrit permet d'écarter de manière suffisante le masque du visage pour enlever le casque tout en maintenant les lunettes de protection dans leur position normale sur ce dernier.
La seconde raison concerne l'apparition de buée lorsque le porteur du masque n'effectue pas un déplacement apte à rendre totalement opérationnels, grâce au flux d'air, les dispositifs de ventilation habituels (orifices pratiqués dans l'écran ou dans la monture). La présente invention permet, en écartant le masque du visage, une circulation d'air additionnelle et suffisante entre le masque et le visage pour contrer efficacement la formation de buée sur la face intérieure de l'écran de protection.

Le dispositif selon l'invention permet de remédier à ces inconvénients. Il comporte, selon une première caractéristique, deux éléments symétriques placés de part et d'autre du casque. Chaque élément est constitué d'un volet rectangulaire, d'une hauteur légèrement supérieure à celle du bandeau du masque, pivotant autour d'un axe. Cet axe solidarise le volet à un support, en contact direct avec le casque et n'opérant aucun mouvement lors de l'actionnement du volet.

Pour la facilité de compréhension, nous décrirons le fonctionnement d'un des deux éléments disposés de part et d'autre du casque, étant entendu que le principe du second est exactement identique.

L'élément est placé entre le bandeau élastique et le casque, à hauteur des tempes, l'axe étant à proximité du bord du casque. L'axe est situé perpendiculairement au bandeau élastique du masque. Le volet, lorsqu'il est plaqué contre le casque vers l'arrière, autorise le placement normal du masque sur celui-ci. Lorsqu'il est actionné d'arrière en avant, le volet vainc la résistance élastique du bandeau pour se retrouver, au terme d'une rotation d'environ 180°, maintenu par le bandeau vers l'avant, en porte-à-faux par rapport au bord du casque. L'extrémité libre du volet fait alors office de nouveau point d'appui du masque. Lorsque chacun des volets est actionné de part et d'autre du casque, le masque se retrouve maintenu vers l'avant, à une distance approximative égale à la différence entre la longueur du volet d'une part et la distance séparant l'axe de rotation du bord du casque d'autre part.

Pour actionner chacun des volets, ceux-ci sont munis d'un levier pratiqué dans le prolongement de leur longueur de sorte qu'il dépasse du bord du bandeau élastique soit en bas de celui-ci, soit en haut. Lorsque le volet est positionné vers l'avant, ledit levier prend appui sur la monture du masque pour constituer une butée qui interrompt le mouvement du volet et assure un maintien stable à la monture.

Pour faciliter le mouvement du volet, son extrémité libre est munie d'un rouleau monté sur axe permettant au bandeau du masque de rouler librement sur celui-ci lorsqu'il est actionné dans un sens ou dans l'autre.

Selon des modes particuliers de réalisation
- La partie de chacun des deux éléments latéraux constituant le support peut être façonnée dans un matériau souple permettant d'épouser au plus prêt le galbe du casque
- La partie de chacun des deux éléments latéraux constituant le support peut être façonnée, à son extrémité antérieure, pour être fixée et prendre appui sur le rebord du casque afin d'éviter tout mouvement intempestif du support lorsque le volet est manipulé.
- La partie de chacun des deux éléments latéraux constituant le support peut être munie, à son extrémité postérieure, d'un système de fixation non inclus dans ce brevet (adhésif ou autre) afin d'éviter tout mouvement intempestif du support.Les supports et volets constituant chaque élément du dispositif peuvent être muni de rebords permettant de maintenir le bandeau du masque bien en place sur le dispositif, que le volet soit en position arrière ou avant.
- Le support et le volet peuvent constituer une seule pièce, une zone de moindre épaisseur formant l'articulation
   Les supports et volets constituant le dispositif peuvent être munis d'un ergot et d'une encoche de sorte que le volet reste plaqué contre le support en position arrière lorsque aucun masque n'est posé sur le casque
- La partie de chacun des deux éléments latéraux constituant le support peut être une pièce faisant partie intégrante du casque et façonnée de sorte à accueillir directement le volet qui s'y articulerait
- Chacun des deux éléments latéraux du dispositif peut être une pièce faisant partie intégrante du casque et façonnée de sorte à reproduire le principe de fonctionnement du dispositif adaptable décrit plus haut

Les dessins annexés illustrent l'invention.
La figure 1 représente l'élément gauche du dispositif

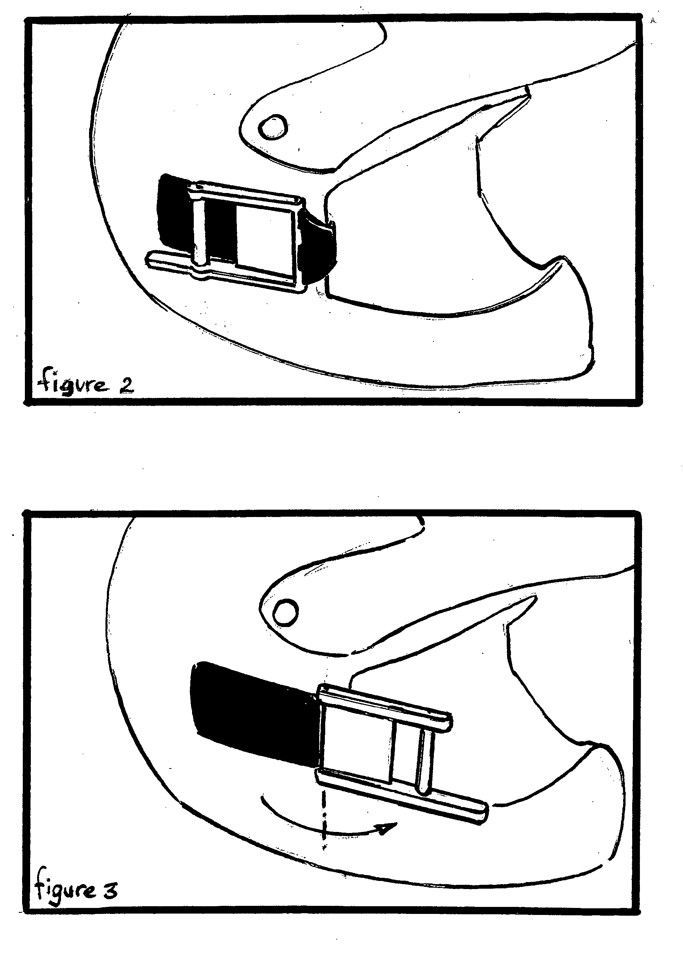

Les figures 2 et 3 (photos) représentent le prototype de l'élément droit du dispositif

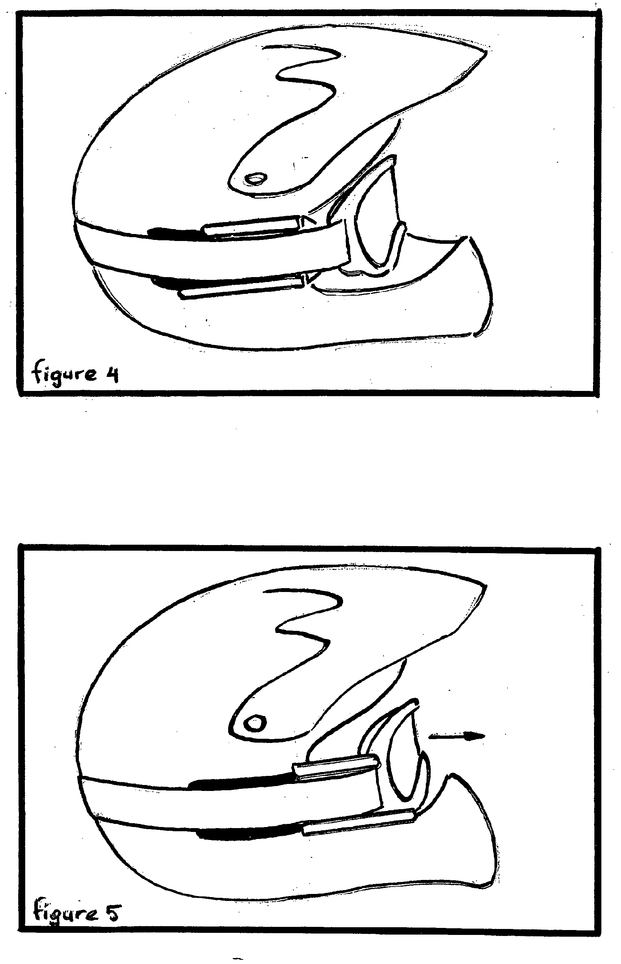

La figure 4 (photo) illustre l'effet du dispositif sur un masque lorsque le volet est en position arrière
La figure 5 (photo) illustre l'effet du dispositif sur un masque lorsque le volet est en position arrière

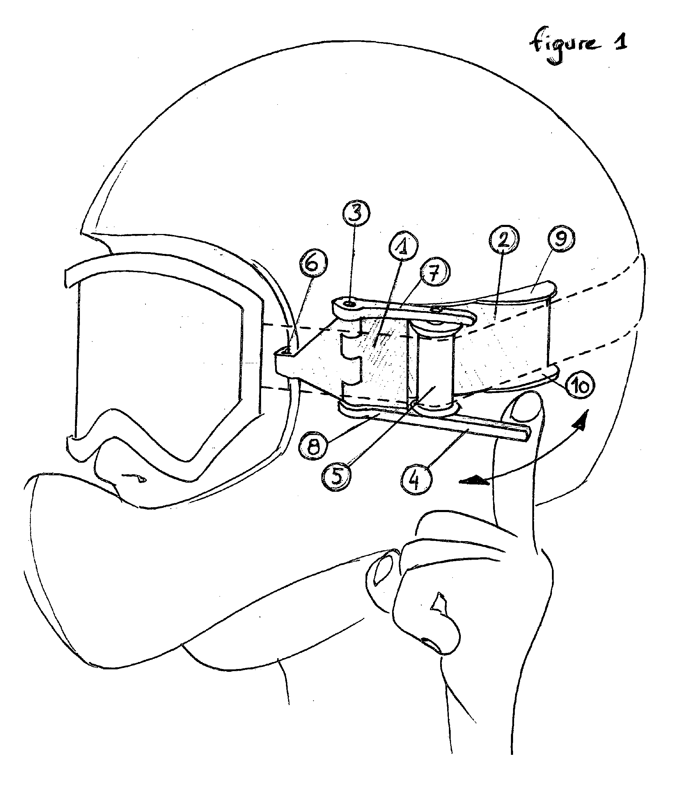

En référence à la figure 1, l'élément gauche du dispositif présente un volet (1) articulé sur un support (2) par le biais d'un axe (3) et pourvu d'un levier (4) permettant d'actionner le volet et servant de butée sur le masque lorsque celui-ci est rabattu vers l'avant. Le volet est équipé d'un rouleau (5) monté sur axe pour faciliter le mouvement du volet. Le support, dans sa partie antérieure, est muni d'un crochet (6) lui permettant d'être fixée au casque et de prendre appui sur le rebord de ce dernier. Des rebords (7, 8, 9, 10) permettent de maintenir le bandeau du masque bien en place sur le dispositif, que le volet soit en position arrière ou avant.

## Revendications

1. Dispositif pour éloigner et maintenir éloigné du visage un masque de protection oculaire à bandeau élastique lorsqu'il est porté sur un casque, **caractérisé en ce qu'**il comporte deux éléments symétriques composés chacun d'un volet (1) articulé sur un support (2) par le biais d'un axe (3) et pourvu d'un levier (4) permettant d'actionner le volet et servant de butée sur le masque lorsque le volet est rabattu vers l'avant.

2. Dispositif selon la revendication 1 **caractérisé en ce que** le volet est équipé d'un rouleau (5) monté sur axe pour faciliter le mouvement du volet

3. Dispositif selon la revendication 1 ou la revendication 2 **caractérisé en ce que** le support, dans sa partie antérieure, est muni d'un crochet (6) lui permettant d'être fixée au casque et de prendre appui sur le rebord de ce dernier.

4. Dispositif selon l'une quelconque des revendications précédentes **caractérisé en ce que** les supports peuvent être façonnés dans un matériau souple et disposer de dispositifs de fixation afin d'épouser au mieux la courbure du casque

5. Dispositif selon l'une quelconque des revendications précédentes **caractérisé en ce que** les supports et les volets peuvent disposer de rebords permettant de maintenir le bandeau du masque bien en place sur le dispositif, que le volet soit en position arrière ou avant.

6. Dispositif selon l'une quelconque des revendications précédentes **caractérisé en ce que** volet et support peuvent ne constituer qu'une seule pièce, une zone de moindre épaisseur formant l'articulation.

7. Dispositif selon l'une quelconque des revendications précédentes **caractérisé en ce que** supports et volets constituant le dispositif peuvent être munis d'un ergot et d'une encoche de sorte que le volet reste plaqué contre le support en position arrière lorsque aucun masque n'est posé sur le casque

8. Dispositif selon l'une quelconque des revendications précédentes **caractérisé en ce que** Chacun des deux éléments latéraux du dispositif peut être une pièce faisant partie intégrante du casque et façonnée de sorte à reproduire à l'identique le principe de fonctionnement du dispositif

## Patentansprüche

1. Vorrichtung, um vom Gesicht eine Augenschutzmaske mit elastischem Band zu entfernen und entfernt zu halten, wenn sie auf einem Helm getragen wird, **dadurch gekennzeichnet, dass** sie zwei symmetrische Elemente umfasst, die jeweils eine Klappe (1) umfassen, die auf einem Träger (2) mit Hilfe einer Achse (3) gelenkig verbunden ist und mit einem Hebel (4) ausgestattet ist, der ermöglicht, die Klappe zu bedienen und als Anschlag auf der Maske dient, wenn die Klappe nach vorne heruntergeklappt ist.

2. Vorrichtung nach Anspruch 1, **dadurch gekennzeichnet, dass** die Klappe mit einer Rolle (5) ausgestattet ist, die auf einer Achse montiert ist, um die Bewegung der Klappe zu erleichtern.

3. Vorrichtung nach Anspruch 1 oder Anspruch 2, **dadurch gekennzeichnet, dass** der Träger an seinem vorderen Teil mit einem Haken (6) ausgestattet ist, der ermöglicht, dass er an den Helm befestigt wird und auf der Kante dieses aufliegt.

4. Vorrichtung nach einem der vorhergehenden Ansprüche, **dadurch gekennzeichnet, dass** die Träger aus einem flexiblen Material geformt sein können und über Vorrichtungen zur Befestigung verfügen können, um sich an die Krümmung des Helms am besten anzupassen.

5. Vorrichtung nach einem der vorhergehenden Ansprüche, **dadurch gekennzeichnet, dass** die Träger und die Klappen über Kanten verfügen können, die ermöglichen, das Band der Maske auf der Vorrichtung gut in seiner Position zu halten, unabhängig davon, ob sich die Klappe in der hinteren oder in der vorderen Position befindet.

6. Vorrichtung nach einem der vorhergehenden Ansprüche, **dadurch gekennzeichnet, dass** die Klappe und der Träger aus nur einem Stück bestehen können, wobei ein Bereich mit einer geringeren Dichte das Gelenk bildet.

7. Vorrichtung nach einem der vorhergehenden Ansprüche, **dadurch gekennzeichnet, dass** die Träger und die Klappen, die die Vorrichtung bilden, mit einem Ansatz und einem Schlitz ausgestattet sein können, so dass die Klappe gegen den Träger in der hinteren Position gepresst bleibt, wenn keine Maske auf dem Helm angebracht ist.

8. Vorrichtung nach einem der vorhergehenden Ansprüche, **dadurch gekennzeichnet, dass** jedes der zwei seitlichen Elemente der Vorrichtung ein Stück sein kann, das integraler Bestandteil des Helms ist und so hergestellt ist, dass es das Funktionsprinzip der Vorrichtung identisch reproduziert.

## Claims

1. A device for separating, and maintaining a distance between, a person's face and an elastic headband eye protection mask worn on a helmet, **characterized in that** it includes two symmetric elements each consisting of a flap (1) hinged on a support (2) by means of an axis (3) and fitted with a lever (4) enabling to actuate the flap and serving as a stop on the mask when the flap is folded forwards.

2. The device according to claim 1 **characterized in that** the flap is equipped with a roller (5) mounted on an axis to facilitate the movement of the flap.

3. The device according to claim 1 or claim 2 **characterized in that** the support is fitted, in the anterior part thereof, with a hook (6) enabling it to be fixed to the helmet and to rest on the flange of the latter.

4. The device according to any one of the preceding claims **characterized in that** the supports can be shaped in a flexible material and fitted with fixing devices to better match the curvature of the helmet.

5. The device according to any one of the preceding claims **characterized in that** the supports and the flaps may have flanges enabling to hold the headband of the mask in place on the device, whether the flap is in the front or rear position.

6. The device according to any one of the preceding claims **characterized in that** the flap and the support may be formed as a single piece, a zone of lesser thickness forming the joint.

7. The device according to any one of the preceding claims **characterized in that** the supports and flaps constituting the device may be fitted with a lug and a notch so that the flap remains pressed against the support in the rear position when no mask is placed on the helmet.

8. The device according to any one of the preceding claims **characterized in that** each of the two side members of the device may be a piece forming an integral part of the helmet and shaped so as to reproduce the same operating principle of the device.
